# EUROPÄISCHE PATENTSCHRIFT

(11) **EP 0 566 836 B1**
(45) Veröffentlichungstag und Bekanntmachung des Hinweises auf die Patenterteilung: **19.08.1998**
(21) Anmeldenummer: 93102337.8
(22) Anmeldetag: 15.02.1993
(51) Int. Cl.: A61N 5/00, A61N 5/01, A61N 5/02, H01Q 13/20, H01Q 21/29

(54) **Hochfrequenz-Therapiegerät**
High frequency therapeutic device
Appareil de thérapie à haute fréquence

(30) Priorität: 23.04.1992 DE 9205494 U
(43) Veröffentlichungstag der Anmeldung: 27.10.1993
(73) Patentinhaber: MEDIZIN ELEKTRONIK LÜNEBURG KG (GMBH & CO.), D-21339 Lüneburg (DE)
(72) Erfinder: Spethmann, Jens, W-2120 Lüneburg (DE)
(74) Vertreter: Richter, Werdermann & Gerbaulet

(56) Entgegenhaltungen:
- EP-A- 0 334 274
- DE-A- 3 048 682
- DE-U- 7 923 476
- DE-U- 9 205 494
- GB-A- 2 081 559
- NAVY TECHNICAL DISCLOSURE BULLETIN Bd. 2, Nr. 8, 01 August 1977, ARLINGTON US, Seiten 33 - 38 COLEMAN 'Flush Mounting Antenna System'

## Beschreibung

Die vorliegende Erfindung bezieht sich auf das Gebiet der medizinischen Hochfrequenz-Therapie. Sie betrifft ein Hochfrequenz-Therapiegerät, umfassend
a) eine Liege mit einem Liegengestell und einer auf dem Liegengestell angeordneten Liegefläche; und
b) eine Hochfrequenzvorrichtung mit einem HF-Generator und einem an den HF-Generator angeschlossenen HF-Strahler, wobei der HF-Strahler quer zur Liege verläuft und unterhalb der Liegefläche in Längsrichtung der Liege verschiebbar angeordnet ist.

Ein derartiges Hochfrequenz-Therapiegerät ist beispielsweise aus der deutschen Offenlegungsschrift DE-A 27 01 934 oder der deutschen Gebrauchsmusteranmeldung G 79 23 476 U1 bekannt.

Hochfrequenz-Therapiegeräte sind in den verschiedensten Ausführungsformen bekannt. Allen diesen Geräten ist gemeinsam, daß sie mit Hilfe geeigneter HF-Generatoren ein hochfrequentes Feld erzeugen, welches von einem HF-Strahler abgestrahlt wird, in den Körper des Patienten eindringt und dort eine endogene Wärmewirkung auslöst (Diathermie).

Bei einer Ausführungsform derartiger Hochfrequenz-Therapiegeräte ist der HF-Generator in einem ortsfesten Gehäuse untergebracht, während der zugehörige HF-Strahler an einem schwenkbaren Haltearm befestigt und mit dem HF-Generator über entsprechende HF-Leitungen verbunden ist.

Da diese Art des Aufbaues wegen der vielen Gelenke am Haltearm mechanisch und elektrisch aufwendig sowie anfällig ist, ist in den eingangs genannten Druckschriften vorgeschlagen worden, den HF-Generator und den HF-Strahler in einer schlittenartigen Einheit zusammenzufassen, die unterhalb einer Liege in Längsrichtung mittels Rollen am Liegengestell verschiebbar angeordnet ist. Der auf der Liege liegende Patient wird dabei von unten durch die Liegefläche mit der vom HF-Strahler abgegebenen HF-Energie vergleichsweise zum Focusstrahler großflächig bestrahlt.

Während eine solche Hochfrequenz-Diathermie-Liege mechanisch robust, leicht zu bedienen und vergleichsweise einfach aufzubauen ist, ist es aufgrund der speziellen Ausgestaltung und Anordnung des HF-Strahlers mit seinem breiten Abstrahlungsfeld nicht möglich, eng begrenzte, lokale Bestrahlungen, wie sie beispielsweise im HNO-Bereich üblich sind, vorzunehmen.

Es ist daher die Aufgabe der Erfindung, ein Hochfrequenz-Therapiegerät der eingangs genannten Art so weiterzuentwickeln, daß mit geringem Zusatzaufwand lokale Bestrahlungen möglich sind.

Die Aufgabe wird durch die in Anspruch 1 gekennzeichneten Merkmale gelöst. Es ist dabei vorgesehen, daß
c) im Verschiebebereich des HF-Strahlers unterhalb der Liegefläche eine Empfangs-Antenne zur Aufnahme der vom HF-Strahler abgestrahlten Hochfrequenz derart angeordnet ist, daß eine Zuschaltung einer Empfangs-Antenne im Abstrahlungsbereich des HF-Strahlers möglich ist, und
d) die Empfangs-Antenne über eine flexible Koaxialleitung an einen frei beweglichen Fokusstrahler angeschlossen ist.

Der Kern der Erfindung besteht also darin, aus dem Bestrahlungsfeld des HF-Strahlers mittels einer Empfangs-Antenne HF-Leistung abzuzweigen und einem an sich bekannten, frei beweglichen Fokusstrahler zuzuführen, der eine lokale Bestrahlung einzelner Körperpartien erlaubt.

Empfangs- Antenne und Fokusstrahler können dabei einfach als Zusatzeinrichtung an bereits vorhandenen Liegen montiert werden, so daß mit geringem Aufwand die Flexibilität der Bestrahlungseinrichtung wesentlich erhöht wird.

Eine bevorzugte Ausführungsform des erfindungsgemäßen Gerätes zeichnet sich dadurch aus, daß
a) der HF-Strahler als Hohlleiter ausgebildet ist und wenigstens eine Schlitzantenne zum Abstrahlen der Hochfrequenz aufweist;
b) die Empfangs-Antenne ein als Hohlraumresonator ausgebildetes Antennengehäuse umfaßt, welches eine Empfangsöffnung aufweist, die in der bestimmten Stellung der wenigstens einen Schlitzantenne des Hohlleiters direkt gegenüberliegt;
c) die Liege einen verstellbaren Kopfteil aufweist;
d) der wenigstens eine Schlitzantenne auf der der Liegefläche zugewandten Seite des Hohlleiters angeordnet ist;
e) die Antenne zwischen Liegefläche und Hohlleiter im Bereich des Kopfteils der Liege angebracht ist; und
f) eine Reduzierung der HF-Leistung auf die Anforderungen des Focusstrahlers erfolgt.

### 1. Ausführungsform:

Anordnung der Empfangs-Antenne unterhalb des Kopfteiles der Liege. Der HF-Strahler wird unter/an/zur Empfangs-Antenne geführt. Hierdurch ergibt sich der Vorteil, daß die Empfangs-Antenne am Kopfteil ständig montiert bleiben kann, da im Kopfbereich eine Hochfrequenz-Diathermie-Behandlung in der mit dem HF-Strahler ermöglichen, breitstrahlenden Form größerer HF-Leistung ohnehin nicht gewünscht ist.

### 2. Ausführungsform:

Durch eine geeignete Konstruktion wird die Empfangs-Antenne des Focusstrahlers in das Bestrahlungsfeld des HF-Strahlers zugeschaltet.

Weitere Ausführungsformen ergeben sich aus den abhängigen Ansprüchen.

Die Erfindung soll nachfolgend anhand von Ausführungsbeispielen im Zusammenhang mit der Zeichnung näher erläutert werden. Es zeigen
- **Fig. 1**: in schematischer Seitenansicht ein erstes bevorzugtes Ausführungsbeispiel einer Hochfrequenz-Diathermie-Liege nach der Erfindung,
- **Fig**. **2**: einen Querschnitt durch die Liege gemäß Fig.2,
- **Fig. 3**: im Detailausschnitt die Lagerung der verschiebbaren Schlitteneinheit aus Fig.2;
- **Fig**. **4a**: eine Ausführungsform des HF-Strahlers aus Fig. 2 als Hohlleiter mit Öffnungsschlitzen (Schlitzantennen),
- **Fig**. **4b**: eine zu Fig. 4a gehörende Ausführungsform der Empfangs-Antenne,
- **Fig**. **5**: in der Draufsicht eine bevorzugte Befestigungsart für die Empfangs-Antenne bei einem Gerät nach der Erfindung,

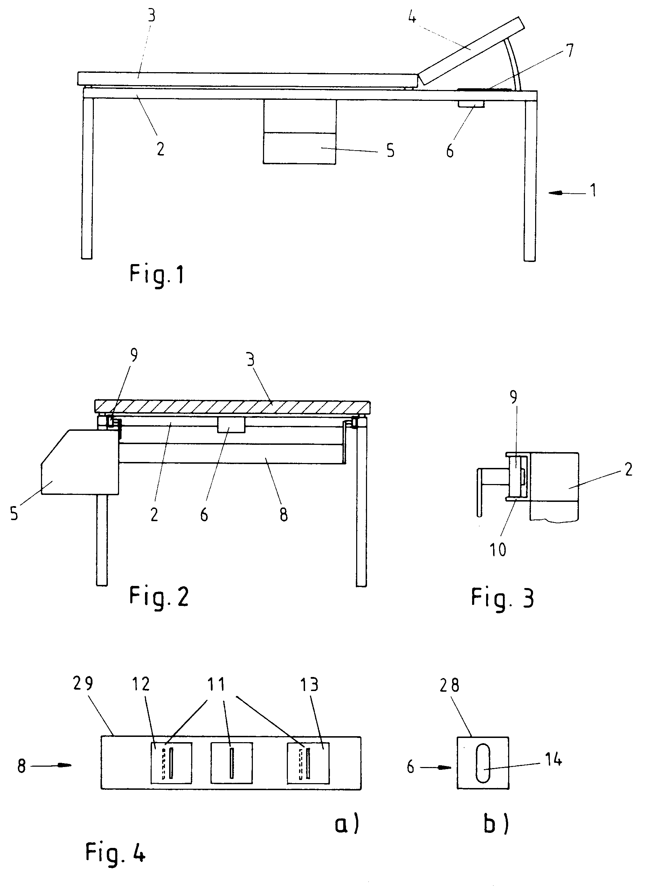

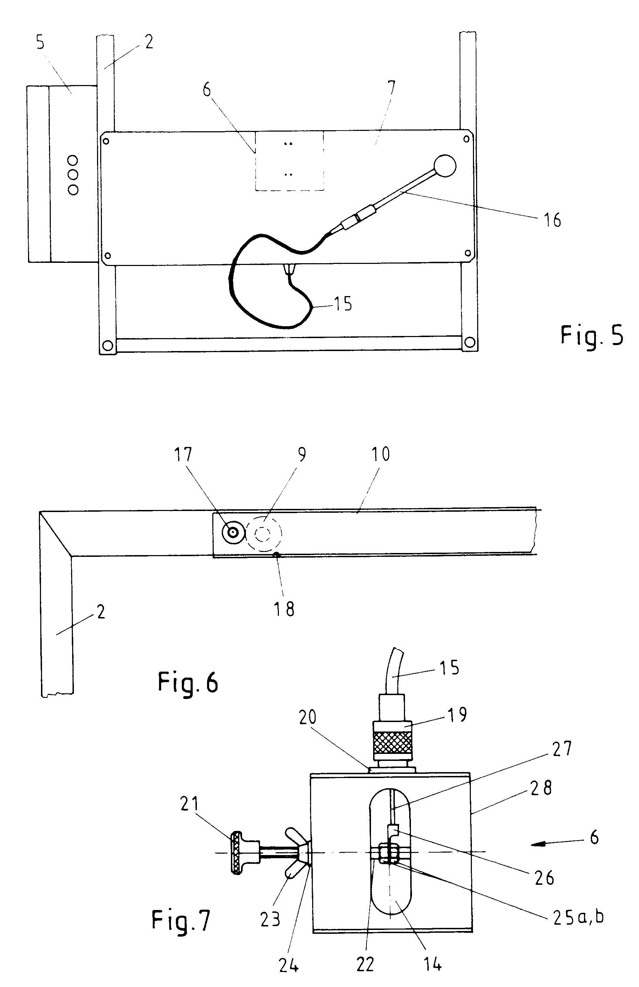

- **Fig. 6**: im Detailausschnitt eine bevorzugte Einrastvorrichtung für den Schlitten nach Fig. 2, und
- **Fig**. **7**: den internen Aufbau der Empfangs-Antenne bei einem bevorzugten Ausführungsbeispiel der Erfindung.

In Fig. 1 ist in der Seitenansicht ein bevorzugtes Ausführungsbeispiel für das erfindungsgemäße Hochfrequenz-Therapiegerät dargestellt. Das Gerät hat die an sich bekannte Form einer Liege 1, die sich aus einem Liegengestell 2 (mit einem Rahmen und Beinen) und einer (in der Regel gepolsterten) Liegefläche 3 zusammensetzt. An wenigstens einem Ende der Liege 1 ist ein Kopfteil 4 vorgesehen, welches z.B. zur besseren Lagerung des Patienten aufgestellt werden kann.

Unterhalb der Liegefläche 3 ist am Rahmen des Liegengestells 2 eine Schlitteneinheit aus einem HF-Generator 5 und einem daran angeschlossenen (in Fig. 1 nicht sichtbaren, aber in Fig. 2 dargestellten) HF-Strahler 8 in Längsrichtung der Liege verschiebbar angeordnet. Der HF-Generator, der zugleich die notwendigen Bedienelemente enthält, erzeugt eine Hochfrequenz, die über den HF-Strahler 8 nach oben durch die Liegefläche 3 hindurch an den darauf liegenden Patienten abgegeben wird. Der Schlitten kann dabei zwischen Kopfende und Fußende der Liege 1 beliebig verschoben werden, so daß grundsätzlich alle Körperteile eines Patienten bestrahlt werden können. Insoweit gleicht die dargestellte den bereits bekannten Liegen.

Unterhalb des Kopfteils 4 ist nun zusätzlich eine Empfangs-Antenne 6 angeordnet, die vorzugsweise auf der Unterseite eines Trägerbleches 7 befestigt ist, welches seinerseits auf dem Rahmen zwischen Liegefläche 3 und Liegengestell 2 angebracht ist. Auf diese Weise kann eine bereits vorhandene Liege auf Dauer mit der zusätzlichen Empfangs-Antenne ausgerüstet werden, ohne daß die normale Funktion in irgendeiner Weise beeinträchtigt wird.

In einer bestimmten Stellung des Schlittens, wenn nämlich der HF-Strahler 8 im Kopfteil der Liege 1 direkt unterhalb der Empfangs-Antenne 6 positioniert ist, nimmt die Empfangs-Antenne aus dem Strahlungsfeld des HF-Strahlers 8 HF-Energie auf, die dann (siehe Fig. 5) über eine flexible Koaxialleitung 15 einem frei beweglichen Focusstrahler 16 zur lokalen Bestrahlung des Patienten zugeführt werden kann. Der Focusstrahler 16 besteht dabei üblicherweise aus einem Handgriffteil und einem Strahlerteil, wie es zur Behandlung im HNO-Bereich verwendet wird.

Die Lagerung des aus HF-Generator 5 und HF-Strahler 8 bestehenden Schlittens erfolgt bevorzugt in der in Fig. 2 bzw. Fig. 3 dargestellten Weise: An den Längsinnenseiten des Rahmens des Liegengestells 2 sind wenigstens zwei parallel und in Längsrichtung der Liege 1 verlaufende Führungsschienen 10 angebracht, in denen der Schlitten mittels Rollen 9 geführt wird. Um ein Einrasten des Schlittens in derjenigen Stellung zu ermöglichen, in welcher der HF-Strahler 8 genau unter der Empfangs-Antenne 6 positioniert ist, sind gemäß Fig. 6 innerhalb der Führungsschienen 10 in deren im Kopfteil 4 liegenden Endbereich als Anschlag für die Rollen 9 Gummipuffer 17 sowie davorliegende von den Rollen 9 überrollbare Nocken 18 vorgesehen: Sobald die vorderen Rollen des Schlittens die Nocken 18 überrollt haben, werden sie in der in Fig. 6 gestrichelt eingezeichneten Position von den Gummipuffern 17 gestoppt und durch die Nocken 18 am unbeabsichtigten Zurückrollen gehindert.

Der HF-Strahler 8 ist gemäß Fig. 4a vorzugsweise als ein Hohlleiter 29 ausgebildet, der auf seiner Oberseite eine Mehrzahl von Schlitzantennen 11 aufweist, durch welche im Normalfall die HF-Leistung auf den Patienten abgestrahlt werden kann. Die Empfangs-Antenne 6 besteht gemäß Fig. 4b entsprechend aus einem einen abgestimmten Hohlraumresonator bildenden Antennengehäuse 28, in welches auf der Unterseite eine längliche Empfangsöffnung 14 eingebracht ist, die in Form und Abmessung mit wenigstens einem der Schlitzantenne 11 des Hohlleiters 29 korrespondiert. Sofern nur eine (in Fig. 4a der mittlere) Sende-Schlitzantenne für die Einkopplung in die Empfangs-Antenne 6 benötigt wird, können die übrigen Sende-Schlitzantennen bei Benutzung des Focusstrahlers 16 durch entsprechende, verschiebbare Schieberplatten 12,13 geschlossen werden.

Im Zusammenhang mit dem Betrieb des Focusstrahlers 16 kann der HF-Generator 5 vorteilhafterweise in gewohnter Weise benutzt werden, da die Leistung aufgrund der bei dieser Anordnung auftretenden Verluste ohnehin in einem (durch die abstimmbare HF-Ankopplung veränderbaren) Verhältnis in der Größenordnung von etwa 10:1 reduziert wird. Unter diesen Voraussetzungen ergibt sich bei einer im Normalfall vom HF-Generator 5 abgegebenen Leistung von 250 Watt im Focusstrahler 16 eine HF-Leistung von 25 Watt.

Die Empfangs-Antenne 6 selbst hat vorzugsweise den in Fig. 7 im Detail dargestellten Aufbau: Die Empfangsöffnung 14 ist im Antennengehäuse 28 als länglicher Schlitz ausgebildet. Innerhalb des Antennengehäuses 28 ist quer zur Empfangsöffnung 14 ein elektrisch leitender Antennenstab 22 angeordnet, welcher in Stabrichtung verschiebbar ist. Seitlich am Antennengehäuse 28 ist ein Anschluß für die Koaxialleitung 15 vorgesehen, der vorzugsweise als HF-Koaxial-Buchse 20 ausgebildet ist. Der Antennenstab 22 selbst ist ein Verbindungsstück 27 mit der HF-Koaxial-Buchse 20 elektrisch verbunden.

Als Antennenstab 22 wird mit Vorteil eine Gewindestange aus Messing verwendet, welche von außen durch eine Wand des Antennengehäuses 28 in den Hohlraum reicht und in einer Wand eingelassenen Nietmutter 24 einschraubbar gelagert wird. Zum Betätigen des Antennenstabes 22 ist am außerhalb des Antennengehäuses 28 liegenden Ende der Gewindestange eine Rändelmutter 21 angebracht. Der Antennenstab 22 kann mittels der Rändelmutter in seiner im Hohlraum liegenden Länge verändert und nach einer so vorgenommenen Abstimmung der Empfangs-Antenne in einer bestimmten Stellung durch eine außerhalb der Nietmutter 24 vorgesehene Kontermutter 23 fixiert werden. Die Kontermutter hat dabei wegen der besseren Handhabbarkeit vorzugsweise die Form einer Flügelmutter.

Als elektrischer Abgriff ist in dem unterhalb der Empfangsöffnung 14 liegenden Bereich der Gewindestange mittig ein mit zwei Muttern 25a,b verlöteter Lötkabelschuh 26 auf der Gewindestange angeordnet, in welchen Lötkabelschuh 26 der Mittelleiter der folgenden Koaxial-Buchse eingelötet ist. Die zum Focusstrahler 16 führende, herkömmliche Koaxialleitung 15 ist an ihrem antennenseitigen Ende mit einem zur HF-Koaxial-Buchse 20 passenden HF-Koaxial-Stecker 19 angeschlossen, so daß die Verbindung zwischen Focusstrahler 16 und Empfangs-Antenne 6 jederzeit leicht gelöst werden kann.

Insgesamt ergibt sich mit der Erfindung eine Hochfrequenz-Diathermie-Liege, die ohne größere Umbauten auch für die lokale Bestrahlung, z.B. im HNO-Bereich, eingesetzt werden kann.

### Bezugszeichenliste:

- Liege: 1
- Liegengestell: 2
- Liegefläche: 3
- Kopfteil: 4
- HF-Generator: 5
- Empfangs-Antenne: 6
- Trägerblech: 7
- HF-Strahler: 8 (Hohlleiter bzw. HF-Strahler)
- Rolle: 9
- Führungsschiene: 10
- Sendeantenne (Schlitzantenne): 11
- Schieberplatte: 12,13
- Empfangsöffnung: 14
- Koaxialleitung: 15
- Focusstrahler: 16
- Gummipuffer: 17
- Nocken: 18
- HF-Koaxial-Stecker: 19
- HF-Koaxial-Buchse: 20
- Rändelmutter: 21
- Antennenstab: 22
- Kontermutter: 23
- Nietmutter: 24
- Mutter: 25a,b
- Lötkabelschuh: 26
- Koaxial-Mittelleiter: 27
- Antennengehäuse: 28
- Hohlleiter: 29

## Patentansprüche

1. Hochfrequenz-Therapiegerät, umfassend
a) eine Liege (1) mit einem Liegengestell (2) und einer auf dem Liegengestell (2) angeordneten Liegefläche (3), und
b) eine Hochfrequenzvorrichtung mit einem HF-Generator (5) und einem an den HF-Generator (5) angeschlossenen HF-Strahler (8), wobei der HF-Strahler (8) quer zur Liege (1) verläuft und unterhalb der Liegefläche (3) in Längsrichtung der Liege (1) verschiebbar angeordnet ist,
dadurch gekennzeichnet, daß
c) im Verschiebebereich des HF-Strahlers (8) unterhalb der Liegefläche (3) eine Empfangs-Antenne (6) zur Aufnahme der vom HF-Strahler (8) abgestrahlten Hochfrequenz derart angeordnet ist, daß bei einer bestimmten Stellung des HF-Strahlers (8) die Empfangs-Antenne (6) im Abstrahlungsbereich des HF-Strahlers (8) liegt, und
d) die Empfangs-Antenne (6) über eine flexible Koaxialleitung (15) an einen frei beweglichen Focusstrahler (16) angeschlossen ist.

2. Hochfrequenz-Therapiegerät nach Anspruch 1, dadurch gekennzeichnet, daß
a) der HF-Strahler (8) als Hohlleiter (29) ausgebildet ist und wenigstens eine Schlitzantenne (11) zum Abstrahlen der Hochfrequenz aufweist, und
b) die Empfangs-Antenne (6) ein als Hohlraumresonator ausgebildetes Antennengehäuse (28) umfaßt, welches eine Empfangsöffnung (14) aufweist, die in der bestimmten Stellung der wenigstens einen Sendeantenne (11) des Hohlleiters (29) direkt gegenüberliegt.

3. Hochfrequenz-Therapiegerät nach Anspruch 2, dadurch gekennzeichnet, daß
a) die Liege (1) einen ggf. verstellbaren Kopfteil (4) aufweist,
b) die wenigstens eine Sendeantenne (11) auf der der Liegefläche (3) zugewandten Seite des Hohlleiters (29) angeordnet ist, und
c) die Empfangs-Antenne (6) zwischen Liegefläche (3) und Hohlleiter (29) im Bereich des Kopfteils (4) der Liege (1) angebracht ist.

4. Hochfrequenz-Therapiegerät nach Anspruch 3,
dadurch gekennzeichnet,
daß die Empfangs-Antenne (6) auf der Unterseite eines Trägerbleches (7) angebracht ist, welches einerseits auf der Oberseite des Liegengestells (2) zwischen Liegengestell (2) und Liegefläche (3) befestigt ist.

5. Hochfrequenz-Therapiegerät nach Anspruch 4,
dadurch gekennzeichnet, daß
a) der HF-Generator (5) und der HF-Strahler (8) einen Schlitten bilden, welcher in wenigstens zwei auf der Innenseite des Liegengestells (2) in Längsrichtung verlaufenden Führungsschienen (10) mittels Rollen (9) verschiebbar gelagert ist, und
b) innerhalb der Führungsschienen (10) in deren im Kopfteil (4) liegenden Endbereich als Anschlag für die Rollen (9) Gummipuffer (17) sowie davorliegende von den Rollen (9) überrollbare Nocken (18) vorgesehen sind, derart, daß der Schlitten mit dem HF-Strahler (8) nach dem Überrollen der Nocken (18) in der bestimmten Stellung unterhalb der Empfangs-Antenne (6) einrastet.

6. Hochfrequenz-Therapiegerät nach einem der Ansprüche 2 bis 5,
dadurch gekennzeichnet, daß
a) die Empfangsöffnung (14) im Antennengehäuse (28) als länglicher Schlitz ausgebildet ist,
b) innerhalb des Antennengehäuses (28) quer zur Empfangsöffnung (14) ein elektrisch leitender Antennenstab (22) angeordnet ist, welcher in Stabrichtung verschiebbar ist,
c) am Antennengehäuse (28) seitlich ein Anschluß für die Koaxialleitung (15) vorgesehen ist, und
d) der Antennenstab (22) über ein Verbindungsstück (27) mit dem Anschluß elektrisch verbunden ist.

7. Hochfrequenz-Therapiegerät nach Anspruch 6,
dadurch gekennzeichnet, daß
a) der Antennenstab (22) als Gewindestange ausgebildet ist, welche von außen durch eine Wand des Antennengehäuses (28) in den Hohlraum reicht und in einer in die Wand eingelassenen Nietmutter (24) gelagert wird,
b) zum Verstellen des Antennenstabes (22) am außerhalb des Antennengehäuses (28) liegenden Ende der Gewindestange eine Rändelmutter (21) angebracht ist,
c) zum Fixieren des Antennenstabes (22) außerhalb der Nietmutter (24) eine Kontermutter (23) in Form einer Flügelmutter vorgesehen ist,
d) als Abgriff in dem unter der Empfangsöffnung (14) liegenden Bereich der Gewindestange ein mit wenigstens einer Mutter (25a,b) verbundener Lötkabelschuh (26) auf der Gewindestange angeordnet ist, in welchen Lötkabelschuh (26) das Verbindungsstück (27) eingelötet ist, und
e) als Anschluß eine HF-Koaxial-Buchse verwendet wird.

8. Hochfrequenz-Therapiegerät nach einem der Ansprüche 1 bis 7,
dadurch gekennzeichnet, daß
a) in dem HF-Strahler (8) eine Vielzahl von Sendeantennen (11) vorgesehen sind,
b) nur bestimmte dieser Sendeantennen für die Abstrahlung auf die Empfangs-Antenne (6) vorgesehen sind, und
c) die übrigen Sende-Schlitz-Antennen im Bedarfsfall durch darüberliegende Schieberplatten (12,13) verschließbar sind.

## Claims

1. High-frequency therapeutic appliance comprising
a) a couch (1) with a couch frame (2) and a couch surface (3) disposed on the couch frame (2), and
b) a high-frequency device with an HF facility (5) and an HF radiator (8) connected to the HF generator, wherein the HF radiator (8) proceeds obliquely to the couch (1) and underneath the couch surface (3) in the longitudinal direction of the couch (1) and is arranged so as to be displaceable,
**characterized in that**
c) within the displacement range of the HF radiator (8), below the couch surface (3), a receiving antenna (6) for receiving the high frequency emitted by the HF radiator (8) is disposed in such a way that, in a specific position of the HF radiator (8), the receiving antenna (6) is located within the radiating range of the HF radiator (8), and
d) the receiving antenna (6) is, via a flexible coaxial transmission line (15), connected to a freely movable focal radiator (16).

2. High-frequency therapeutic appliance according to Claim 1,
**characterized in that**
a) the HF radiator (8) is constructed in the form of a waveguide (29) and possesses at least one slot antenna (11) for emitting the high frequency, and
b) the receiving antenna (6) comprises an antenna housing (28) constructed in the form of a cavity resonator, which possesses a receiving aperture (14) which, in the specific position, is located directly opposite the at least one transmitting antenna (11) of the waveguide (29).

3. High-frequency therapeutic appliance according to Claim 2,
**characterized in that**
a) the couch possesses one possibly adjustable head portion (4),
b) which has at least one transmitting antenna (11) disposed on the side of the waveguide (29) that faces the couch surface (3), and
c) the receiving antenna (6) is mounted between the couch surface (3) and the waveguide (29) within the region of the head portion (4) of the couch (1).

4. High-frequency therapeutic appliance according to Claim 3,
**characterized in that**
the receiving antenna (6) is fitted to the underside of a carrier plate (7), which is attached on the one side of the top side of the couch frame (2) between the couch frame (2) and the couch surface (3).

5. High-frequency therapeutic appliance according to Claim 4,
**characterized in that**
a) the HF generator (5) and the HF radiator (8) constitute a carriage which is displaceably supported in at least two guide rails (10) by means of rollers, which carriage or slide proceeds on the inside of the couch frame (2) in the longitudinal direction, and
b) in that, within the guide rails (10), in their terminal region located in the head portion (4), as stop for the rollers (9), rubber buffers (17) as well as cams (18) located therebefore which can be overrun by the rollers (9) are provided in such a way that the carriage with the HF radiator (8), after having overrrun the cams (18), the carriage with the HF radiator (8) lockingly engages in the specific position underneath the receiving antenna (6).

6. High-frequency therapeutic appliance according to any of Claims 2 to 5,
**characterized in that**
a) the receiving aperture (14) on the antenna housing (28) is constructed in the form of an elongated slot;
b) within the antenna housing (28), obliquely to the receiving aperture (14), an electrically conductive antenna rod (22) is mounted, which is displaceable in the direction of the rod;
c) laterally on the antenna housing (28), a connection for the coaxial transmission line (15) is provided, and
d) the antenna rod (22) is electrically connected to the connection by means of a connecting piece (27).

7. High-frequency therapeutic appliance according to Claim 6,
characterized in that
a) the antenna rod (22) is constructed in the form of a threaded rod which, from the outside, reaches through a wall of the antenna housing (28) into the cavity and is supported in a rivet nut (24) embedded in the wall;
b) a knurled nut (21) is mounted on the end of the threaded rod located outside the antenna housing (28) for the adjustment of the antenna rod (22);
c) a locknut (23) possessing the configuration of a butterfly nut is provided outside the rivet nut (24) for the fixation of the antenna rod (22);
d) as a tap within the area located below the receiving aperture (14) of the threaded rod, a soldering cable terminal (26) connected with at least one nut (25a, b) is disposed on the threaded rod, the connecting piece (27) being soldered into the soldering cable terminal (26), and
c) an HF coaxial socket is employed as connection.

8. High-frequency therapeutic appliance according to any of Claims 1 to 7,
**characterized in that**
a) a plurality of transmitting antennas (11) is provided in the HF radiator (8);
b) only specific ones of these transmitting antennas are provided for the emission on to the receiving antennas (6), and
c) the remaining transmitting slot antennas, if required, can be closed by sliding plates (12, 13) located thereabove.

## Revendications

1. Appareil de thérapie à haute fréquence, comprenant
a) un lit (1) avec un bâti de lit (2) et une couchette (3) disposée sur le bâti de lit (2), et
b) un dispositif haute fréquence avec un générateur haute fréquence (5) et un radiateur haute fréquence (8) raccordé au générateur haute fréquence (5), le radiateur haute fréquence (8) étant transversal par rapport au lit (1) et étant disposé déplaçable en dessous de la couchette (3) dans le sens longitudinal du lit (1),
caractérisé en ce
c) qu'une antenne de réception (6) pour capter la haute fréquence rayonnée par le radiateur haute fréquence (8) est placée dans la zone de déplacement du radiateur haute fréquence (8) en dessous de la couchette (3) de telle manière que, pour une position définie du radiateur haute fréquence (8), l'antenne de réception (6) se trouve dans la zone de rayonnement du radiateur haute fréquence (8) et
d) l'antenne de réception (6) est raccordée par une conduite coaxiale flexible (15) à un radiateur focal (16) mobile librement.

2. Appareil de thérapie à haute fréquence selon la revendication 1, caractérisé en ce que
a) le radiateur haute fréquence (8) est configuré comme un guide creux (29) et présente au moins une antenne fendue (11) pour rayonner la haute fréquence et
b) l'antenne de réception comprend un bâti d'antenne (28), configuré comme résonateur creux, qui présente une ouverture de réception (14) qui, dans la position définie, est située directement en face de l'antenne d'émission (11) qui existe au moins du guide creux (29).

3. Appareil de thérapie à haute fréquence selon la revendication 2,
caractérisé en ce que
a) le lit présente une partie pour la tête (4) éventuellement réglable,
b) l'antenne d'émission (11) qui existe au moins est placée sur le côté du guide creux (29) qui est tourné vers la couchette (3) et
c) l'antenne de réception (6) est disposée entre la couchette (3) et le guide creux (29) dans la zone de la partie pour la tête (4) du lit (1).

4. Appareil de thérapie selon la revendication 3,
caractérisé en ce
que l'antenne de réception (6) est disposée sur la face inférieure d'une tôle de support (7) qui est fixée d'une part sur la face supérieure du bâti du lit (2) entre le bâti du lit (2) et la couchette (3).

5. Appareil de thérapie à haute fréquence selon la revendication 4,
caractérisé en ce que
a) le générateur haute fréquence (5) et le radiateur haute fréquence (8) forment un coulisseau qui est positionné déplaçable au moyen de galets (9) dans au moins deux rails de guidage (10) qui sont sur la face intérieure du bâti de lit (2) dans le sens longitudinal et
b) des butoirs de caoutchouc (17) sont prévus à l'intérieur des rails de guidage (10) dans leur zone d'extrémité qui se situe dans la partie pour la tête (4) ainsi que des ergots (18) roulables par les galets (8) et situés devant les butoirs en caouchouc de manière que le coulisseau s'enclenche avec le radiateur haute fréquence (8) après avoir roulé sur les ergots (18) dans la position définie en dessous de l'antenne de réception (6).

6. Appareil de thérapie à haute fréquence selon l'une des revendications 2 à 5,
caractérisé en ce que
a) l'ouverture de réception (14) est configurée dans le bâti d'antenne (28) comme une fente allongée,
b) un bâton d'antenne (22) électroconducteur est placé à l'intérieur du bâti d'antenne (28) transversalement par rapport à l'ouverture de réception (14), bâton d'antenne qui est déplaçable dans le sens du bâton,
c) un raccord pour la conduite coaxiale (15) est prévu latéralement sur le bâti d'antenne (28) et
d) le bâton d'antenne (22) est relié électriquement par une pièce de jonction (27) au raccord.

7. Appareil de thérapie à haute fréquence selon la revendication 6,
caractérisé en ce que
a) le bâton d'antenne (22) est configuré comme une tige filetée qui entre de l'extérieur à travers une paroi du bâti d'antenne (28) dans l'espace creux et qui est positionnée dans un écrou à river (24) encastré dans la paroi,
b) pour régler le bâton d'antenne (22), un écrou moleté (21) est fixé à l'extrémité de la tige filetée qui est située à l'extérieur du bâti d'antenne (28),
c) pour fixer le bâton d'antenne (22) à l'extérieur de l'écrou à river (24) un contre-écrou (23) en forme d'écrou à oreilles est prévu,
d) une cosse de câble à braser (26), reliée à au moins un écrou (25a, b), est placée sur la tige filetée comme prise dans la zone de la tige filetée qui se trouve en dessous de l'ouverture de réception (14), cosse de câble à braser (26) dans laquelle la pièce de jonction (27) est brasée et
e) une douille coaxiale haute fréquence est utilisée comme raccord.

8. Appareil de thérapie à haute fréquence selon l'une des revendications 1 à 7,
caractérisé en ce
a) qu'une multitude d'antennes d'émission (11) sont prévues dans le radiateur haute fréquence (8),
b) que seulement certaines de ces antennes d'émissions sont prévues pour le rayonnement sur l'antenne de réception (6) et
c) les autres antennes fendues d'émission peuvent être fermées au besoin par des plaques coulissantes (12, 13) qui se trouvent au-dessus.
